# EUROPEAN PATENT SPECIFICATION

(11) **EP 3 589 374 B1**
(45) Date of publication and mention of the grant of the patent: **19.05.2021**
(21) Application number: 18712463.1
(22) Date of filing: 27.02.2018
(51) Int. Cl.: A61Q 11/00, A61K 9/00, A61K 9/06

(54) **CHLORHEXIDINE-BASED MOUTHWASH PREVENTING TEETH-STAINING**
MUNDSPÜLUNG AUF BASIS VON CHLORHEXIDIN ZUR VERHINDERUNG VON ZAHNVERFÄRBUNG
BAIN DE BOUCHE À BASE DE CHLORHEXIDINE EMPÊCHANT LA COLORATION DES DENTS

(30) Priority: 28.02.2017 IT 201700022601
(43) Date of publication of application: 08.01.2020
(73) Proprietor: Unifarco S.p.A., 32035 Santa Giustina (BL) (IT)
(72) Inventor: BARATTO, Giovanni, 32035 Santa Giustina BL (IT); RIGANO, Luigi Mario, 32035 Santa Giustina BL (IT)
(74) Representative: Perani & Partners S.p.A.
(86) International application number: PCT/IB2018/051233
(87) International publication number: WO 2018/158683

(56) References cited:
- EP-A1- 1 340 490
- EP-A1- 2 774 481
- EP-A1- 2 939 710
- FR-A1- 2 972 347

## Description

### FIELD OF THE INVENTION

The present invention relates to a chlorhexidine-based mouthwash for use in the prevention and control of plaque formation.

### PRIOR ART

Chlorhexidine is a known antibacterial agent used medically and cosmetically in many applications.

From a chemical-structural point of view, it is a poly-biguanide (bisbiguanide) with cationic properties, typically used in the form of dichlorhydrate, diacetate and digluconate salts.

The molecule consists of two symmetrical chlorophenyl rings and two biguanidic groups that connect to a central hexamethylene ring.

At physiological pH, the chlorhexidine salts dissociate and release in solution the positively charged chlorhexidine cation, whose ability to interact with the bacterial cell wall, negatively charged, gives the molecule bactericidal properties. (Dr. Gurbani Kaur, Dr. Archana Singh, Dr. Kaustubh P. Patil, Dr. Gopalakrishnan, Dr. Abhishek Singh Nayyar, Dr. Sachin Deshmukh; Chlorhexidine: a cationic bisbiguanide, membrane active drug in periodontal medicine, structure- advantages and associated adverse effects, a brief communication. World journal of pharmacy and pharmaceutical sciences, Volume 4, Issue 07, 370-392, Review Article ISSN 2278 - 4357).

Used as a disinfectant in the gynaecological, urological, dermatological and surgery field, chlorhexidine is widely used in periodontal medicine, as an active ingredient for dental products aiming at plaque prevention and control. (Setu Mathur, Tanu Mathur, Rahul Shrivastava, Rohit Khatri, Chlorhexidine: The gold standard in chemical plaque control, Natl. J. Physiol. Pharm. Pharmacol., 2011; 1 (2): 45-50).

The use of chlorhexidine for prolonged periods, however, causes the browning of the tissues of the oral cavity (teeth and tongue), an effect that deters the prolonged use of chlorhexidine-based mouthwashes, despite the incredibly effective bacteriostatic activity.

US 2004/0265246 discloses an oral hygiene mouthwash comprising chlorhexidine and ascorbic acid, characterized in that it also comprises sodium metabisulphite and optionally an alkali metal fluoride. Said mouthwash, as well as preventing the formation of dental plaque and caries, avoids the browning of the tissues of the oral cavity.

### SUMMARY OF THE INVENTION

The applicant has now found a composition comprising chlorhexidine, which is effective in maintaining oral hygiene and reduces the browning of the teeth even more effectively than shown by known prior art products.

An object of the present invention is a composition comprising chlorhexidine or a salt thereof, ascorbyl glucoside and ortho-, meta- or para-hydroxyacetophenone for topical-oral use.

A further object of the present application is the use of the aforesaid composition for the prevention, and preferably the control, of the formation of plaque and dental caries.

### DESCRIPTION OF THE FIGURES

Figure 1: measurements of parameter b* obtained for test 1 (chlorhexidine digluconate 0.2%)
Figure 2: measurements of parameter b* obtained for test 2 (chlorhexidine digluconate 0.2% + ascorbic acid 0.2%)
Figure 3: measurements of parameter b* obtained for test 3 (chlorhexidine digluconate 0.2% + ascorbyl glucoside 0.2%)
Figure 4: measurements of parameter b* obtained for test 4 (chlorhexidine digluconate 0.2% + ascorbyl glucoside 0.2% + hydroxyacetophenone 0.1%).

### DETAILED DESCRIPTION OF THE INVENTION

For the purposes of the present invention, composition for topical-oral use means a composition for external use, suitable for application on the tissues of the oral cavity.

The oral cavity includes the two front thirds of the tongue, the gums, the inner surface of the cheeks and lips, the lower part of the mouth under the tongue (the oral floor), the upper part of the mouth (the hard palate) and the area beyond the wisdom teeth (the retro molar trigon).

Ascorbyl glucoside is a modified form of vitamin C, in which the hydroxyl group of the ascorbic acid C2 is stabilized with a glucose molecule.

Originally developed as a pro-drug of vitamin C, ascorbyl glucoside in vivo is metabolized by the enzyme alpha-glucosidase, which releases ascorbic acid in vivo, breaking the covalent bond with the glucose molecule.

Ascorbyl glucoside is generally used in cosmetics, in the formulation of creams and lotions, for clearing skin spots and freckles, as well as for improving skin tone in general.

Besides being used to obtain a brighter and more even skin tone, ascorbyl glucoside is also used to counteract the effects of skin aging and as a sunscreen in UV skin protection products.

As well as ascorbyl glucoside, also hydroxyacetophenone is originally known for being used in the preparation of cosmetic products, as a dose-dependent antioxidant, an anti-irritant and a preservative.

In the cosmetic field, it is common practice to indicate the ingredients of a composition by using the INCI (International Nomenclature of Cosmetic Ingredients) system, i.e. the list of cosmetic ingredients expressed according to a standard nomenclature.

According to the INCI system, the hydroxyacetophenone ingredient corresponds to the only para- form of the compound, para-hydroxyacetophenone (http://ec.europa.eu/growth/tools-databases/cosing/index.cfm?fuseaction=search.details_v2&id = 90948; International Cosmetic Ingredient Dictionary and Handbook, Sixteenth Edition 2016), which currently is the only form of hydroxyacetophenone commercially available for the production of cosmetic preparations.

Without being bound to any theory, it is believed that the reaction of chlorhexidine with extrinsic factors in the oral cavity is at the base of the formation of brownish spots.

These factors are diet-related dentinal film, proteins, carbohydrates, tin and iron pigmented sulphides, protein denaturation products, chromogens and polyphenols.

The use of an anti-oxidant could promote a decrease in the interaction between the chlorhexidine molecule and the main extrinsic factors mentioned above.

The Applicant has found by means of in vitro tests that the use of ascorbyl glucoside, which is more stable than the simple ascorbic acid, in addition to hydroxyacetophenone, allows obtaining a solution in which the two antioxidants enhance one another's action by working in synergy.

In this way, the obtained antioxidant system (antioxidant network) can actively act on the discoloration induced by the use of chlorhexidine.

The composition object of the present invention preferably comprises chlorhexidine in an amount of between 0.02% and 2% (w/w), ascorbyl glucoside in an amount of between 0.08% and 5% (w/w) and hydroxyacetophenone in an amount of between 0.02% and 2% (w/w).

The composition object of the present invention includes e.g. chlorhexidine in an amount of between 0.1% and 1% (w/w), ascorbyl glucoside in an amount of between 0.1% and 1% (w/w) and hydroxyacetophenone in an amount of between 0.1% and 1% (w/w).

The composition object of the present invention includes e.g. chlorhexidine in an amount of between 0.16% and 0.24% (w/v), ascorbyl glucoside in an amount of between 0.16% and 0.24% (w/v) and hydroxyacetophenone in an amount of between 0.08% and 0.12% (w/v).

Chlorhexidine is preferably in salt form and, even more preferably, said salt is selected among a chlorhexidine digluconate, diacetate or dichlorhydrate salt.

For the purposes of the present invention, hydroxyacetophenone means the compound in an isomeric form selected among para-hydroxyacetophenone, meta-hydroxyacetophenone and ortho-hydroxyacetophenone or mixtures thereof.

According to a preferred embodiment, the composition comprises para-hydroxyacetophenone or ortho-hydroxyacetophenone or mixtures thereof.

Without being bound to any scientific theory, it is believed that para- and ortho-hydroxyacetophenone are most effective in the formation of an antioxidant system thanks to the position of the hydroxyl group on the aromatic ring, which allows the electron delocalization between two limit forms of resonance (mesomerie).

Both for the para- and for the ortho-hydroxyacetophenone, the electronic cloud is delocalized between two electronegative atoms, namely between the oxygen of the hydroxyl group and the one of the acetyl group, which are species predisposed to the attraction of the bonding electrons towards the nucleus.

The meta-form, although capable of performing an anti-oxidant action, is less favoured, since the electronic cloud is distributed between an electronegative atom, the oxygen of the hydroxyl group and the aromatic ring carbons, which are less electronegative than the oxygen and therefore less predisposed to electronic delocalization.

Even more preferably, the composition comprises para-hydroxyacetophenone.

It should be noted that the purified para-hydroxyacetophenone, commercially available, might sometimes contain reaction by-products and/or synthetic intermediates as impurities.

Among these impurities, there is ortho-hydroxyacetophenone, which may be present in amounts at least equal to 0.1% by weight out of the total weight of para-hydroxyacetophenone.

The composition object of the present invention is preferably used in the prevention of dental plaque and caries.

The composition can be formulated in the form of mouthwash, toothpaste, gel.

### EXPERIMENTAL SECTION

The results obtained by treating teeth with mouthwashes containing 0.2% chlorhexidine digluconate are described below.

The results made it possible to check whether the teeth immersed in the mouthwashes maintain their natural coloration unchanged and to ascertain the effectiveness of the formulation in avoiding the chromatic changes commonly due to the use of chlorhexidine-containing products.

### Materials and methods

The teeth, obtained from extractions, were treated for 15 minutes in 2% sodium hypochlorite, washed with distilled water and dried.

The teeth were immersed in the following aqueous solutions:
- test 1: 0.2% chlorhexidine digluconate solution;
- test 2: 0.2% chlorhexidine digluconate solution, 0.2% ascorbic acid and sodium metabisulphite;
- test 3: 0.2% chlorhexidine digluconate solution and 0.2% ascorbyl glucoside;
- test 4: 0.2% chlorhexidine digluconate solution, 0.2% ascorbyl glucoside and 0.1% hydroxyacetophenone.

The teeth were stored in the mouthwashes for 4 weeks at room temperature.

Photographs with flashes were taken at the teeth before immersing them in the solutions and after 1 and 2 weeks of immersion.

A colorimetric analysis was then performed to verify the extent of the tooth colouring alteration.

The Konica Minolta Chroma Meter CR-400 colorimeter, which performs measurements on a small area (8 mm in diameter), was used for the measurements.

Measurements were made on the photo shoots of the teeth treated with the different solutions (at time 0, after 1 and 2 weeks).

In this test, every single measurement derives from the average of 3 measurements, which is automatically provided by the instrument. For each photograph, 3 measurements were taken.

The used detection system is called CIELab and the measured parameters correspond to the Cartesian coordinates L*, a*, b*:
- L* = indicates brightness, expressed as a percentage (0 for black and 100 for white)
- a* = indicates the green-red colour component, with values from -60 to +60
- b* = indicates the blue-yellow colour component, with values from -60 to +60

The following are the measurements obtained for the b* parameter, which is more related to the purpose of the study.

The increase in parameter b* corresponds to an increase in yellowing.

### Results

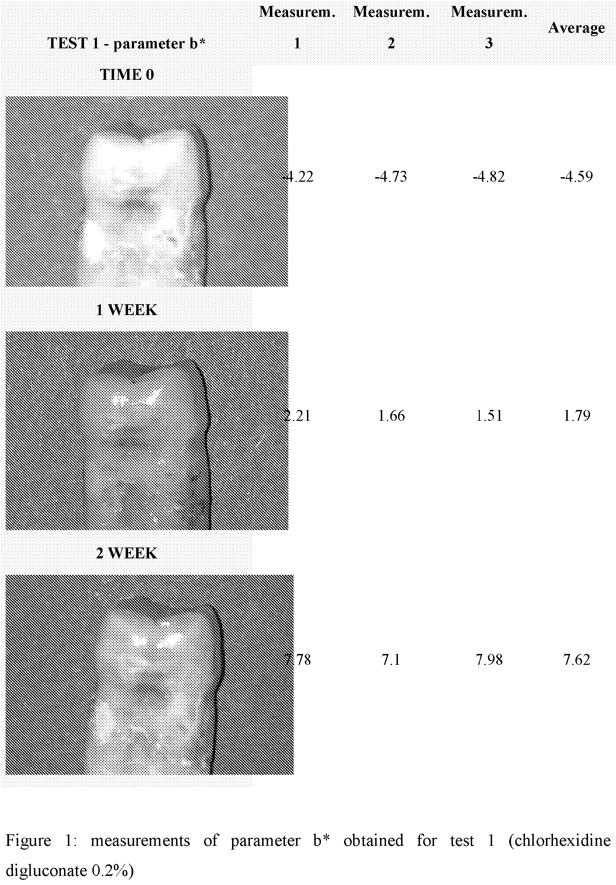

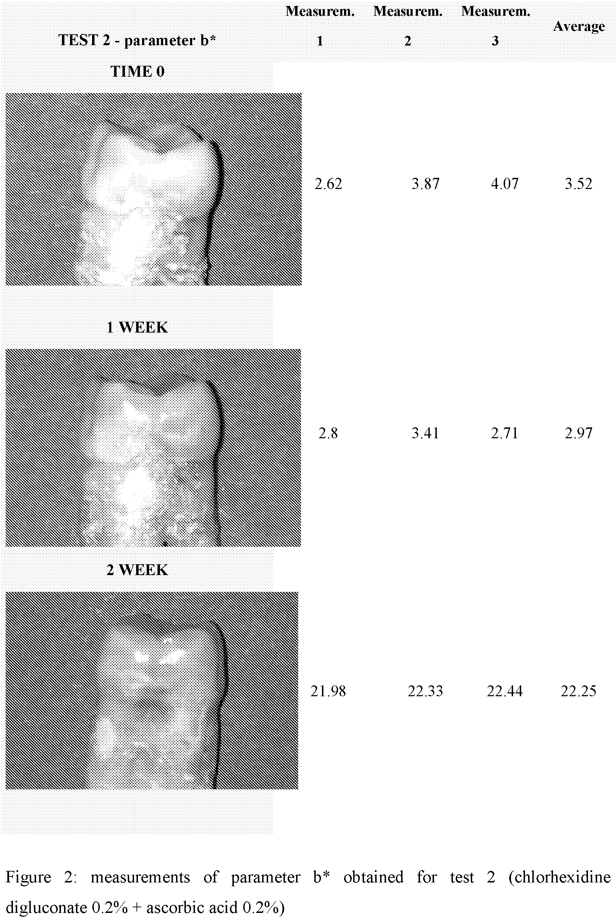

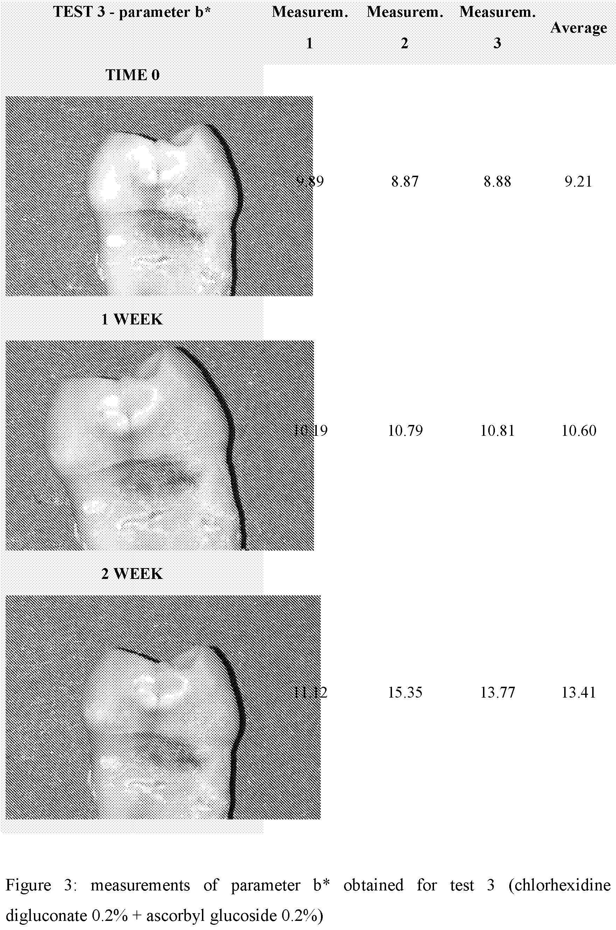

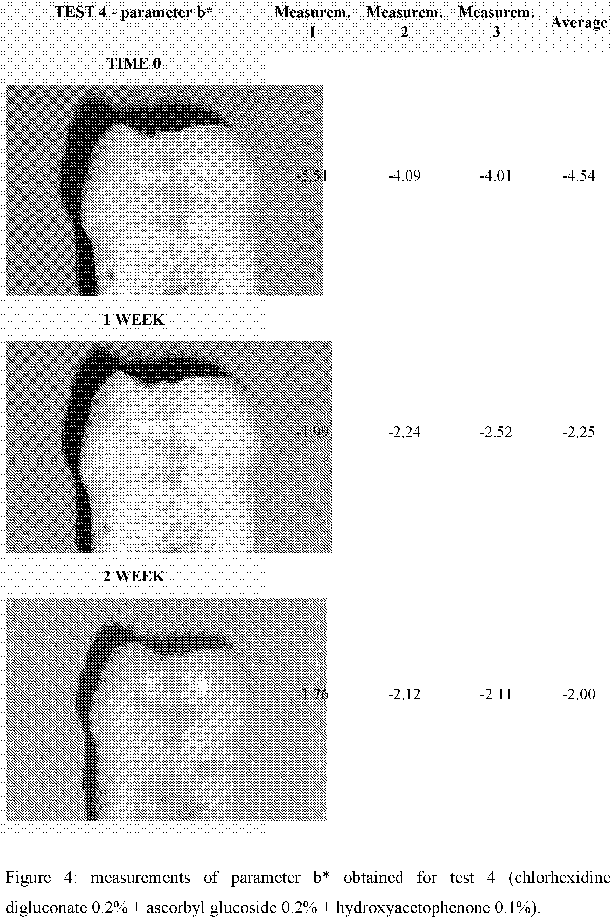

Figures 1-4 show the values obtained the measurements carried out by means of a colorimeter. Here enclosed the photo shoots on which the analyses were performed.

The following table shows the variations of parameter b* between time 0 and final time (2 weeks) for each of the four tests.

**Table 5: variation parameter b***

| **Variation parameter b*** | |
|---|---|
| **TEST 1** | 12.21 |
| **TEST 2** | 18.73 |
| **TEST 3** | 4.20 |
| **TEST 4** | 2.54 |

By applying the t-test function, the values measured for test 4 can be compared with those measured for the other tests after 2 weeks, thus establishing whether there is a significant difference between the values. With p ≤ 0.05, the difference between the values is deemed relevant.

The results are shown in the following table.

**Table 6: t-test**

| |
|---|
| TEST 4 vs TEST 1: p = 0.0007 |
| TEST 4 vs TEST 2: p = 0.0001 |
| TEST 4 vs TEST 3: p = 0.0075 |

### Conclusions

The measurements performed by means of a colorimeter indicate that the yellowing of the tooth immersed in an aqueous solution of chlorhexidine digluconate 0.2% + ascorbyl glucoside 0.2% + hydroxyacetophenone 0.1% (Test 4) is lower than the one shown by the other teeth at the end of the treatment.

With the application of the t-test function it was also shown that the yellowing measured for the tooth of test 4 is significantly different from the yellowing of the other teeth.

Examples of formulations comprising the composition object of the present patent application are reported below for illustrative and non-limiting purposes.

### EXAMPLES

### Mouthwash

Humectants (Sorbitol) 1 - 7%
Allantoin 0.01 - 2%
Anionic/amphoteric/non-ionic surfactants (Laureth 23) 0.01 - 10%
HYDROXYACETOPHENONE 0.1 - 1%
CHLORHEXIDINE DIGLUCONATE 0.1 - 1%
ASCORBYL GLUCOSIDE 0.1 - 1%
Flavour 0.1 - 1%
Sweeteners (xylitol, sucralose) 0.01 - 0.2%
Water qs to 100

### Toothpaste

Water qs to 100
Hydrotropes (glycerine, sorbitol, xylitol) 3 - 40%
Polymer viscosity enhancers (xanthan gum, guar gum, cellulose derivatives) 0.1-3%
Mineral abrasive (silica) 15 - 50%
Foaming agent (anionic/amphoteric surfactants) 0.02 - 3%
Preservatives 0-1%
Flavour 0.01-2%
HYDROXYACETOPHENONE 0.1 - 1%
CHLORHEXIDINE DIGLUCONATE 0.1 - 1%
ASCORBYL GLUCOSIDE 0.1 - 1%

### Gel

Water qs to 100
Polymer viscosity enhancers (xanthan gum/guar, cellulose derivatives) 0.1 - 4% Sweetener 0.1-0.5%
Preservatives 0-1%
Flavour 1-2%
HYDROXYACETOPHENONE 0.1 - 1%
CHLORHEXIDINE DIGLUCONATE 0.1 - 1%
ASCORBYL GLUCOSIDE 0.1 - 1%

## Claims

1. Composition comprising chlorhexidine or a salt thereof, ascorbyl glucoside and hydroxyacetophenone, wherein hydroxyacetophenone is selected among the isomeric forms para-hydroxyacetophenone, meta-hydroxyacetophenone, ortho-hydroxyacetophenone or mixtures thereof, and said composition is for topical-oral use.

2. Composition according to claim 1, wherein the chlorhexidine or its salt thereof is contained in an amount comprised between 0.02% and 2% (w/w), ascorbyl glucoside is contained in an amount comprised between 0.08% and 5% (w/w) and hydroxyacetophenone is contained in an amount comprised between 0.02% and 2% (w/w).

3. Composition according to any one of claims 1 and 2, wherein said chlorhexidine salt is selected among a digluconate, diacetate or dichlorhydrate salt.

4. Composition according to any one of claims 1 to 3 for use in preventing dental plaque and caries.

5. Composition according to any one of claims 1 to 3 in the form of a mouthwash, toothpaste, gel.

## Patentansprüche

1. Zusammensetzung, umfassend Chlorhexidin oder ein Salz davon, Ascorbylglucosid und Hydroxyacetophenon, wobei Hydroxyacetophenon aus den isomeren Formen Para-Hydroxyacetophenon, Meta-Hydroxyacetophenon, Ortho-Hydroxyacetophenon oder Mischungen davon ausgewählt ist, und die Zusammensetzung zur topischen oralen Verwendung bestimmt ist.

2. Zusammensetzung nach Anspruch 1, wobei das Chlorhexidin oder sein Salz davon in einer Menge zwischen 0,02% und 2% (Gew./Gew.), Ascorbylglucosid in einer Menge zwischen 0,08% und 5% (Gew./Gew.) und Hydroxyacetophenon in einer Menge zwischen 0,02 % und 2% (Gew./Gew.) enthalten ist.

3. Zusammensetzung nach einem der Ansprüche 1 und 2, wobei das Chlorhexidinsalz aus einem Digluconat-, Diacetat- oder Dichlorhydratsalz ausgewählt ist.

4. Zusammensetzung nach einem der Ansprüche 1 bis 3 zur Verwendung bei der Prävention von Zahnbelag und Karies.

5. Zusammensetzung nach einem der Ansprüche 1 bis 3 in Form eines Mundwassers, einer Zahnpasta, eines Gels.

## Revendications

1. Composition comprenant de la chlorhexidine ou un sel de celle-ci, du glucoside d'ascorbyle et de l'hydroxyacétophénone, où l'hydroxyacétophénone est choisie parmi les formes isomères para-hydroxyacétophénone, méta-hydroxyacétophénone, ortho-hydroxyacétophénone ou des mélanges de celles-ci, et ladite composition est destinée à une utilisation topique-orale.

2. Composition selon la revendication 1, où la chlorhexidine ou son sel est contenu en une quantité comprise entre 0,02% et 2% (p/p), le glucoside d'ascorbyle est contenu en une quantité comprise entre 0,08% et 5% (p/p) et l'hydroxyacétophénone est contenue en une quantité comprise entre 0,02% et 2% (p/p).

3. Composition selon l'une quelconque des revendications 1 et 2, où ledit sel de chlorhexidine est choisi parmi un sel de digluconate, de diacétate ou de dichlorhydrate.

4. Composition selon l'une quelconque des revendications 1 à 3, destinée à être utilisée pour prévenir la plaque dentaire et les caries.

5. Composition selon l'une quelconque des revendications 1 à 3 sous forme de bain de bouche, dentifrice, gel.
